(19) Europäisches Patentamt
European Patent Office
Office européen des brevets

(11) Publication number: 0 392 802 A2

(12) EUROPEAN PATENT APPLICATION

(21) Application number: 90303849.5

(51) Int. Cl.[5]: A61K 31/17, A61K 31/275

(22) Date of filing: 10.04.90

The title of the invention has been amended (Guidelines for Examination in the EPO, A-III, 7.3).

(30) Priority: 11.04.89 GB 8908063

(43) Date of publication of application: 17.10.90 Bulletin 90/42

(84) Designated Contracting States: AT BE CH DE DK ES FR GB GR IT LI LU NL SE

(71) Applicant: Beecham Group p.l.c.
SB House Great West Road
Brentford Middlesex TW8 9BD(GB)

(72) Inventor: Evans, John Morris, Beecham Pharmaceuticals
Coldharbour Road, The Pinnacles
Fourth Avenue, Harlow, Essex CM19 5AD(GB)
Inventor: Stemp, Geoffrey, Beecham Pharmaceuticals
Coldharbour Road, The Pinnacles
Fourth Avenue, Harlow, Essex CM19 5AD(GB)
Inventor: Hadley, Michael Stewart, Beecham Pharmaceuticals
Coldharbour Road, The Pinnacles
Fourth Avenue, Harlow, Essex CM19 5AD(GB)

(74) Representative: Rutter, Keith et al
Smith Kline Beecham, Corporate Patents,
Great Burgh, Yew Tree Bottom Road
Epsom Surrey KT18 5XQ(GB)

# (54) Preparation of urea analogues and their use in bronchial, cerebrovascular and neuronal disorders.

(57) A method for the treatment of disorders of the respiratory system and/or for the treatment of cerebrovascular disorders and/or neuronal degenerative disorders which method comprises the administration, to a human or non-human mammal in need of such treatment, of an effective, non-toxic amount of a compound of formula (I), or a pharmaceutically acceptable salt thereof, or a pharmaceutically acceptable solvate thereof:

$$R_1-NH\overset{\overset{R_2}{\|}}{C}NHR_3 \qquad (I)$$

wherein:

$R_1$ is an aryl group substituted with up to three substituents selected from the group consisting of: CN, $NO_2$, $CF_3$, halogen, $C_{2-12}$ alkyl and $C_{2-12}$ alkyl carbonyl;

$R_2$ is S or NCN; and

$R_3$ is an alkyl group or an aryl group; certain compounds having pharmacological activity, a process to prepare such compounds and pharmaceutical compositions comprising such compounds.

EP 0 392 802 A2

Xerox Copy Centre

# NOVEL METHOD AND COMPOUNDS

This invention relates to a novel method of treatment, to certain novel compounds, to processes for the preparation of such compounds and to their use as therapeutic agents.

U.S. Patents No. 4623662 and 4387106 (American Cyanamid Co.) disclose various N′-disubstituted-N-aryl-thioureas as anti-cholesterol agents.

European Patent Number 0,022,958 (Bayer) discloses compounds of formula (A)

$R^a{}_1R^a{}_4N\text{-}CX\text{-}NR^a{}_2R^a{}_3$ (A)

in which:

$R^a{}_1$ and $R^a{}_2$ are hydrogen or optionally-substituted alkyl, cycloalkyl or aralkyl groups;

$R^a{}_3$ and $R^a{}_4$ are optionally-substituted aryl or heteroaryl groups, and

$X^a$ is O, S or NCN;

as compounds useful for inhibiting lipid absorption.

Selected thioureas of formula (A) are also disclosed in European Patent Number 0,049,538.

In J. Med. Chem 1978. Vol. 21, No. 8, pp. 773-781, various N-alkyl-N′-pyridyl-thioureas and N-alkyl-N′-pyridyl N″-cyanoguanidines are shown to have hypotensive activity.

European patent application, publication number 0,354,553 discloses compounds of formula (B):

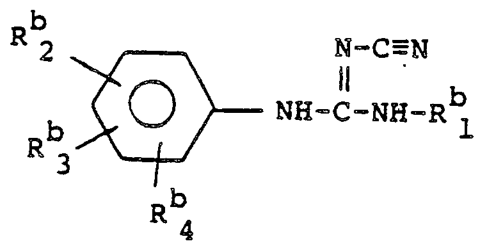

(B)

and tautomers thereof and pharmaceutically acceptable salts, wherein $R^b{}_1$ is alkyl, alkenyl, alkynyl, haloalkyl, cycloalkyl, aryl, arylalkyl or cycloalkylalkyl;

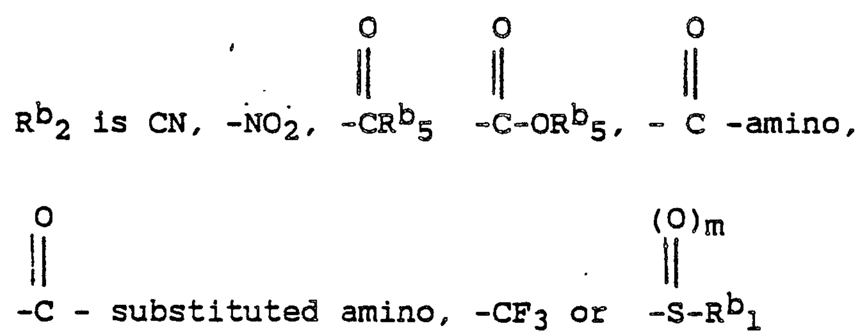

$R^b{}_3$ and $R^b{}_4$ are each independently selected from $R^b{}_2$, hydrogen, alkyl, alkenyl, alkynyl, haloalkyl, halo, alkoxy, -NHalkyl, $-N(alkyl)_2$, -S-alkyl, -O-arylalkyl, -S-arylalkyl or -S-aryl, -O-aryl, -NHarylalkyl, or $R^b{}_2$ and $R^b{}_3$ taken together are a group which forms a ring with the two carbon atoms to which they are attached, which group is selected from:

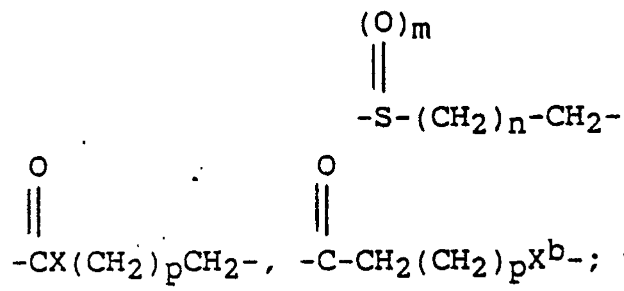

wherein

m = 1 or 2, n = 3-5, p = 2-4, $X^b$ is O, $NR^b_5$, $CH_2$ and $R^b_5$ is hydrogen or $R^b_1$

The compounds of formula (B) are stated to be useful as antihypertensive agents and for the treatment of fibrillation of the heart.

EP 0,354,553 is relevant to the present application only in so far as dictated by Article 54(3) of the European Patent Convention.

The present invention is based on the finding that a group of substituted thioureas and cyanoguanidines are surprisingly indicated as being of potential use in the treatment of disorders of the cardiovascular system and the respiratory system, and in particular as cerebral blood flow enhancers.

Thus these compounds are indicated to have been smooth muscle relaxant activity, and are therefore potentially useful as bronchodilators in the treatment of disorders of the respiratory tract, such as reversible airways obstruction and asthma, and also in the treatment of hypertension. They are also indicated to be of potential use in the treatment of disorders associated with smooth muscle contraction of the gastro-intestinal tract, uterus or the urinary tract including the ureter. Such disorders respectively include irritable bowel syndrome and diverticular disease; premature labour; incontinence; renal cholic and disorders associated with the passage of kidney stones.

These compounds are also indicated to be of potential use in the treatment of cardiovascular disorders such as hypertension, congestive heart failure, angina, peripheral vascular disease and in the treatment and/or prophylaxis of disorders associated with pulmonary hypertension and of disorders associated with right heart failure.

The cerebral blood flow enhancement properties of these compounds are of potential use in the treatment of cerebrovascular disorders, such as multi-infarct dementia and neuronal degenerative disorders associated with learning, memory and cognitive dysfunctions including cerebral senility, senile dementia of the Alzheimer type, age associated memory impairment and certain disorders associated with Parkinson's disease.

Accordingly, the present invention provides a method for the treatment of disorders of the respiratory system and/or for the treatment of cerebrovascular disorders and/or neuronal degenerative disorders which method comprises the administration, to a human or non-human mammal in need of such treatment, of an effective, non-toxic amount of a compound of formula (I), or a pharmaceutically acceptable salt thereof, or a pharmaceutically acceptable solvate thereof:

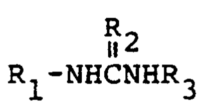

(I)

wherein:

$R_1$ is an aryl group substituted with up to three substituents selected from the group consisting of: CN, $NO_2$, $CF_3$, halogen, $C_{2-12}$ alkyl and $C_{2-12}$ alkyl carbonyl;

$R_2$ is S or NCN; and

$R_3$ is an alkyl group or an aryl group.

In an alternative aspect, the present invention also provides the use of a compound of formula (I), or a pharmaceutically acceptable salt thereof, or a pharmaceutically acceptable solvate thereof, for the manufacture of a medicament for the treatment of disorders of the respiratory system and/or for the treatment of cerebrovascular disorders and/or neuronal degenerative disorders.

The present invention also provides a pharmaceutical composition for the treatment of disorders of the respiratory system and/or for the treatment of cerebrovascular disorders and/or neuronal degenerative disorders which composition comprises a non-toxic pharmaceutically acceptable amount of a compound of formula (I), or a pharmaceutically acceptable salt thereof, or a pharmaceutically acceptable solvate thereof.

In yet a further aspect, the present invention provides a method for the treatment of disorders of the cardiovascular system which method comprises the administration, to a human or non-human mammal in need of such treatment, an effective, non-toxic amount of a compound of the abovedefined formula (I), or a pharmaceutically acceptable salt thereof, or a pharmaceutically acceptable solvate thereof, providing that when $R_1$ represents 3- or 4-cyanophenyl and $R_3$ is 1,2,2-trimethylpropyl or when $R_1$ represents 2,6-dichlorophenyl and $R_3$ is tert. butyl, then $R_2$ is S.

The invention also provides the use of a compound of formula (I), or a pharmaceutically acceptable salt thereof, or a pharmaceutically acceptable solvate thereof, providing that when $R_1$ represents 3- or 4-cyanophenyl and $R_3$ is 1,2,2-trimethylpropyl or when $R_1$ represents 2,6- dichlorophenyl and $R_3$ is tert. butyl, then $R_2$ is S, for the manufacture of a medicament for the treatment of cardiovascular disorders such, as hypertension.

Certain of the compounds of formula (I) are considered to be novel compounds:

The present invention therefore provides a compound of formula (IA), or a salt thereof, or a solvate thereof:

$$R_1-NH\overset{\overset{R_2}{\|}}{C}NHR_3 \qquad \text{(IA)}$$

wherein:

$R_1$ is an aryl group substituted with up to three substituents selected from the group consisting of: CN, $NO_2$, $CF_3$, halogen, $C_{2-12}$ alkyl and $C_{2-12}$ alkyl carbonyl;

$R_2$ is S or NCN; and

$R_3$ is a alkyl group or an aryl group,

provided that when $R_1$ is 4-tert. butyl-phenyl and $R_3$ is phenyl, then $R_2$ is NCN and provided that when $R_1$ represents 3- or 4-cyanophenyl and $R_3$ is 1,2,2-trimethylpropyl or when $R_1$ represents 2,6-dichlorophenyl and $R_3$ is tert. butyl, then $R_2$ is S.

The substituents of $R_1$ may be present at any position on $R_1$, for example the 3- or the 4- positions. The 3-and 4- positions are especially favoured when $R_1$ represents phenyl, in particular the 4- position.

Suitably, $R_1$ is monosubstituted.

Favoured substituents for $R_1$ are CN or halogen such as F.

A preferred substituent for $R_1$ is CN.

A preferred substituent for $R_1$ is F.

Preferred values for $R_1$ include 3-, and especially, 4-cyanophenyl, 3-, and especially, 4-fluorophenyl.

Preferably, $R^2$ represents S.

$R_3$ is preferably an alkyl group, suitably a $C_{1-9}$ alkyl group, for example a $C_{1-6}$, $C_{4-6}$ or a $C_{5-9}$ alkyl group, and at higher carbon numbers is preferably branched. Particular alkyl groups for $R_3$ include branched $C_4$, $C_5$, $C_6$ or $C_7$ alkyl groups, especially branched $C_4$, such as tert. butyl, or branched $C_6$, such as trimethyl propyl for example 1,2,2-trimethyl propyl.

One preferred sub-group of a compound of formula (IA) is a compound of formula (IB):

$$R_1-NH\overset{\overset{R_2}{\|}}{C}NHR_3 \qquad \text{(IB)}$$

wherein:

$R_1$ is phenyl monosubstituted with CN, $NO_2$, $CF_3$, halogen, $C_{2-6}$ alkyl or a $C_{2-6}$ alkyl carbonyl group;

$R_2$ is S or NCN; and

$R_3$ is a $C_{1-9}$ alkyl group or phenyl, provided that when $R_1$ is 4-tert. butyl-phenyl and $R_3$ is phenyl, then $R_2$ is NCN and provided that when $R_1$ represents 3- or 4-cyanophenyl and $R_3$ is 1,2,2-trimethylpropyl or when $R_1$ represents 2,6- dichlorophenyl and $R_3$ is tert. butyl, then $R_2$ is S.

Typical examples of compounds in accordance with formula (IA) are:
(±)-N-4-cyanophenyl-N′-1,2,2-trimethylprop-1-ylthiourea;
(±)-N-3-cyanophenyl-N′-1,2,2-trimethylprop-1-ylthiourea;
(±)-N-4-fluorophenyl-N′-1,2, 2-trimethylprop-1-ylthiourea;
(±)-N″-cyano-N-4-fluorophenyl-N′-1,2,2-trimethylprop-1-ylguanidine; and
N-4-cyanophenyl-N′-t-butylthiourea.

The term 'halogen' refers to fluorine, chlorine, bromine and iodine, especially fluorine and chlorine, and in particular fluorine.

Suitable alkyl groups may be straight or branched chain and may contain up to 12 carbon atoms such as $C_{1-6}$ or $C_{2-6}$ carbon atoms. Suitable $C_{1-6}$ alkyl groups may be methyl, ethyl, n-, or iso-propyl, or n-, sec-, iso- or tert-butyl. Suitable $C_{2-6}$ alkyl groups include ethyl, n-, or iso-propyl, or n-, sec-, iso- or tert-butyl.

Suitable aryl groups include phenyl and naphthyl, but especially phenyl.

The compounds of formula (I) or (IA) or their salts or solvates are preferably in pharmaceutically acceptable or substantially pure form.

By pharmaceutically acceptable form is meant, inter alia, of a pharmaceutically acceptable level of purity excluding normal pharmaceutical additives such as diluents and carriers, and including no material considered toxic at normal dosage levels.

A substantially pure form will generally contain at least 50% (excluding normal pharmaceutical additives), preferably 75%, more preferably 90% and still more preferably 95% of the compound of formula (I) or its salt or solvate.

One preferred pharmaceutically acceptable form is the crystalline form, including such form in a pharmaceutical composition. In the case of salts and solvates the additional ionic and solvent moieties must also be non-toxic.

Examples of a pharmaceutically acceptable salt of a compound of formula (I) or (IA) include the acid addition salts with the conventional pharmaceutical acids, for example, hydrochloric, hydrobromic and phosphoric.

Examples of a pharmaceutically acceptable solvate of a compound of formula (I) or (IA) includes a hydrate thereof.

Certain of the compounds of formula (I) or (IA) have at least one asymmetric centre and may therefore exist in more than one stereoisomeric form, for example when either $R_1$ and/or $R_3$ represents a chiral alkyl group. The invention extends to all stereoisomeric forms of the compounds of formula (I) or (IA) and to mixtures thereof, including racemates.

The present invention also provides a process for the preparation of a compound of formula (IA) in which $R_2$ is S, in which a compound of formula (II)

$R_1' - N = C = S$ (II)

is reacted with a compound of formula (III)

$R_3'NH_2$ (III)

where $R_1'$ and $R_3'$ are respectively $R_1$ and $R_3$ or groups convertible thereto, to form a compound of formula (IC)

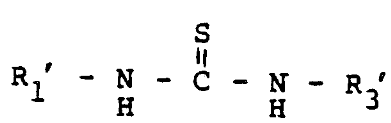

(IC)

and where appropriate or necessary converting $R_1'$ or $R_3'$ to $R_1$ and $R_3$ respectively, and/or forming a salt or solvate.

The reaction is typically carried out in an inert solvent such as dichloromethane, ethers or hydrocarbons, at room temperature. The reaction temperature is not critical.

The present invention also provides on process for forming a compound of formula (IA) in which $R_2$ is NCN, which comprises reacting a compound of formula (IV)

$R_1' - N = C = N - R_3'$ (IV)

with cyanamide, to form a compound of formula (ID)

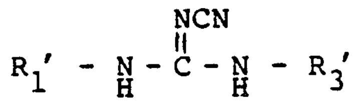

(ID)

and where appropriate or necessary converting $R_1'$ or $R_3'$ to $R_1$ and $R_3$ respectively, and/or forming a salt or solvate.

The reaction is preferably carried out with base catalysis either by mixing the reagents directly or in an inert solvent. A suitable solvent is acetonitrile, or ether, tetrahydrofuran (THF) or dichloromethane. The reaction mixture may be heated to speed up the reaction.

The carbodiimide of formula (IV) may be prepared from a compound of formula (V)

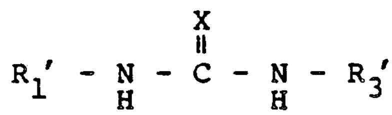

(V)

where $R_1'$ and $R_3'$ are as previously defined and X is S or O [when X is S, then formula (V) includes compounds of formula (I)] by reaction with phosgene to form an intermediate of formula (VI)

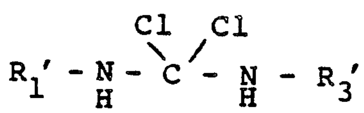

(VI)

which in the presence of a base is dehydrochlorinated to form a compound of formula (IV).

The reaction with phosgene is typically carried out in an inert solvent such as ethers, THF, dichloromethane or hydrocarbons. The temperature is preferably between 0°C and room temperature to maintain the phosgene in liquid form. Alternatively higher temperatures may be used in a pressurised reactor. Suitable bases for the dehydrochlorination include triethylamine or diisopropylethylamine. The same inert solvents may be used at 0°C to room temperature.

Compounds of formula (II) may be prepared by reaction of a compound of formula (VII)

$R_1' - NH_2$ (VII)

with thiophosgene, to form an intermediate of formula (VIII)

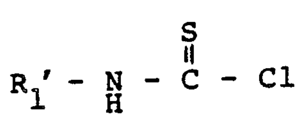

(VIII)

which spontaneously dehydrochlorinates to form a compound of formula (II).

The reaction is carried out in water or a mixture of water and organic solvent, typically at 0°C to room temperature, although heating may be needed in some cases.

The reaction sequence from formula (VII) to formula (II) to formula (I) may also be carried out in the opposite sense starting with a compound of formula (III)

$R_3'NH_2$ (III)

to form a compound of formula (IIA) by reaction with thiophosgene

$R_3'$-N=C=S (IIA)

which is reacted with a compound of formula (VII)

$R_1'$ - $NH_2$ (VII)

to obtain a compound of formula (IA).

Suitable conversions of $R_1'$ to $R_1$ generally include interconversions of the substituents of the group $R_1$.

Suitable conversions of $R_1'$ to $R_1$ and $R_3'$ to $R_3$ may be carried out using any appropriate convention procedure,

Generally $R_1'$ represents $R_1$. Generally $R_3'$ represents $R_3$.

Further information on the preparation of isothiocyanates anlogous to formula (II) and carbodi-imides analogous to formula (IV), and alternative routes to such compounds may be found "Comprehensive Organic Chemistry" Barton and Ollis, Vol. 2, part 8, pages 514-521, and the references cited therein. The same source provides information on preparation of compounds of formula (V) in which X is O, effectively by process analogous to preparation of compounds of formula (I) ($R_2$/X = S) via an isocyanate instead of isothiocyanate.

Further information on conditions appropriate to preparation of thioureas and cyanoguanidines can be derived from the J. Med. Chem. paper acknowledged earlier and the references cited therein.

The compounds of formula (I) or (IA) may be converted into their pharmaceutically acceptable acid addition salts by reaction with the appropriate organic or mineral acids.

Solvates of the compounds of formula (I) or (IA) may be formed by crystallization or recrystallization from the appropriate solvent. For example hydrates may be formed by crystallization or recrystallization from aqueous solutions, or solutions in organic solvents containing water.

Salts or solvates of the compounds of formula (I) or (IA) which are not pharmaceutically acceptable may be useful as intermediates in the production of pharmaceutically acceptable salts or solvates. Accordingly such salts or solvates also form part of this invention.

The compounds of formula (I) or (IA) exist in more than one stereoisomeric form and the processes of the invention produces mixtures thereof. The individual isomers may be obtained by the usual methods, for example asymmetric synthesis, or the use of chiral chromatographic techniques.

Certain intermediates described above are novel compounds and, together with the described processes for their preparation, they form a further aspect of this invention.

The activity of the compounds of formula (I) and (IA) in standard tests indicates that they are of therapeutic utility in the treatment of disorders of the cardiovascular and respiratory system, especially for enhancement of cerebral blood flow.

Accordingly in a further aspect, the present invention also provides a compound of formula (IA), or a pharmaceutically acceptable salt, or a pharmaceutically acceptable solvate thereof, for use as an active therapeutic substance.

The present invention further provides a pharmaceutical composition comprising a therapeutically effective amount of a compound of formula (IA), or a pharmaceutically acceptable salt, or a pharmaceutically acceptable solvate thereof, and a pharmaceutically acceptable carrier.

Such a medicament, and a composition of this invention, may be prepared by admixture of a compound of the invention with an appropriate carrier. It may contain a diluent, binder, filler, disintegrant, flavouring agent, colouring agent, lubricant or preservative in conventional manner.

These conventional excipients may be employed for example as in the preparation of compositions of known agents for these disorders.

The compositions are preferably adapted for oral administration. However, they may be adapted for other modes of administration, for example parenteral administration for patients suffering from heart failure. Other alternative modes of administration include sublingual or transdermal administration. A composition may be in the form of spray, aerosol or other conventional method of inhalation, for treating respiratory tract disorders.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating. Conventional sustained release formulations of the compounds of formula (I) are also envisaged.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight, of the active material, depending on the method of administration.

An effective amount will depend on the relative efficacy of the compound, the severity of the disorder being treated and the weight of the sufferer. However, a unit dose form of a composition of the invention may contain from 0.05 to 500 mg of a compound of the invention and more usually from 0.1 to 50 mg, for example 0.5 to 25 mg such as 0.5, 1, 2, 5, 10, 15 or 20mg. Such compositions may be administered from 1 to 6 times a day, more usually from 1 to 4 times a day, in a manner such that the daily dose is from 0.01 to 25 mg for a per kg body weight and more particularly from 0.1 to 10 mg/kg.

No toxicological effects are indicated at the aforementioned dosage ranges.

The following examples illustrate the invention but do not limit it in any way.

Example 1

(±)-N-4-Cyanophenyl-N′-1,2,2-trimethylprop-1-ylthiourea (E1)

(±) 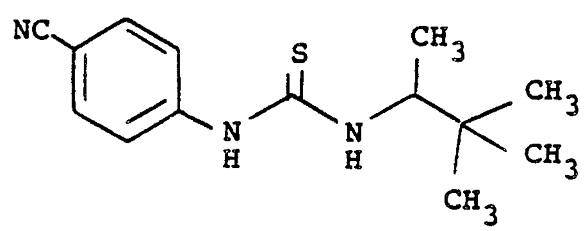 (E1)

4-Aminobenzonitrile (5.9g) was added portionwise to a vigorously stirred mixture of thiophosgene (6.32g), dichloromethane (20 ml) and water (40 ml) at room temperature. After 30 min potassium hydrogen carbonate (10g) in water (50 ml) was added portionwise. After stirring for a further 15 min, the aqueous phase was removed and the organic phase washed with 1N hydrochloric acid (2x50 ml), water (3x50 ml), 5% (w/v) sodium hydrogencarbonate solution (50 ml) then saturated sodium chloride solution. The organic solution was then treated with anhydrous potassium carbonate and 2-amino-3,3-dimethylbutane (6.07g) and the mixture stirred for 16 h at room temperature. The mixture was filtered and the residue washed with chloroform (100 ml). The combined filtrate was washed with 1N hydrochloric acid (3x100 ml), water (2x100

ml), 5% (w/v) sodium hydrogencarbonate solution (100 ml) and then brine (2x100 ml). The organic solution was dried over anhydrous magnesium sulphate, filtered and the solvent was evaporated in vacuo, to give an orange solid. Trituration under ether gave the title compound as a white solid (8.18g); recrystallisation from ethyl acetate - 60-80 petrol gave a sample of m.p. 166-7°C.

270 MHz $^1$H-nmr ($CDCl_3$) $\delta$ 0.93 (s,9H); 1.15 (d, J=7Hz, 3H); 4.29-4.54 (broad signal, 1H); 6.16 (d, J=8Hz, 1H); 7.38 (d, J=8Hz, 2H); 7.7 (d, J=8Hz, 2H); 8.52 (br s, 1H).

Mass spectrum: Found $M^+$ 261.1299; $C_{14}H_{19}N_3S$ requires 261.1300.

| | | | |
|---|---|---|---|
| Analysis: Found: | C, 64.22; | H, 7.21; | N, 16.09. |
| $C_{14}H_{19}N_3S$ requires: | C, 64.33; | H, 7.33; | N, 16.08%. |

## Example 2

(±)-N-3-Cyanophenyl-N′-1,2,2-trimethylprop-1-ylthiourea (E2)

(±) 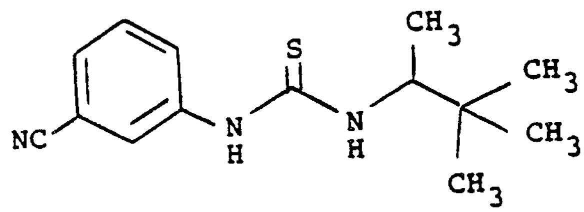 (E2)

The title compound was prepared analogously to the preparation of the 4-cyanophenylthiourea of Example 1, starting from 3-aminobenzonitrile. Recrystallisation from ethyl acetate - 60-80 petrol gave a white solid of m.p. 142-4°C.

270 MHz $^1$H-nmr ($CDCl_3$) $\delta$ 0.98 (s, 9H); 1.15 (d, J=7Hz, 3H); 4.20-4.70 (broad signal, 1H); 5.80-6.30 (br.m, 1H); 7.45-7.84 (m, 4H); 8.20-8.80 (broad signal, 1H). Mass spectrum: Found $M^+$ 261.1300; $C_{14}H_{19}N_3S$ requires 261.1300.

| | | | |
|---|---|---|---|
| Analysis: Found: | C, 64.33; | H, 7.30; | N, 16.02. |
| $C_{14}N_{19}N_3S$ requires: | C, 64.33; | H, 7.33; | N, 16.08%. |

## Example 3

(±)-N-4-Fluorophenyl-N′-1,2,2-trimethylprop-1-ylthiourea (E3)

(±) 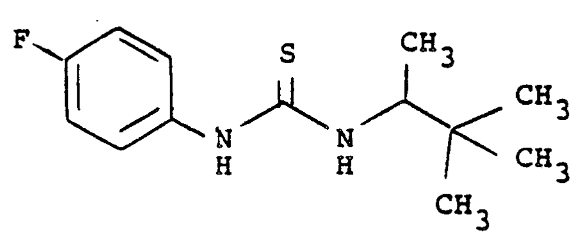 (E3)

The title compound was prepared analogously to the preparation of the 4-cyanophenylthiourea of Example 1, starting from 4-amino-fluorobenzene. Recrystallisation from ethyl acetate - 60:80 petrol gave a white solid of m.p. 148-151°C.

270 MHz $^1$H-nmr ($CDCl_3$) δ, 0.87 (s, 9H); 1.09 (d, J=7Hz, 3H); 4.30-4.49 (m, 1H); 5.73 (d, J=9Hz, 1H); 7.09-7.27 (m, 4H); 8.09 (s, 1H).

| | | | |
|---|---|---|---|
| Analysis: Found: | C, 61.45; | H, 7.55; | N, 10.81. |
| $C_{13}H_{19}N_2SF$ requires: | C, 61.38; | H, 7.35; | N, 11.01%. |

Example 4

(±)-N″-Cyano-N-4-cyanophenyl-N′-1,2,2-trimethylprop-1-ylquanidine (E4)

(±) 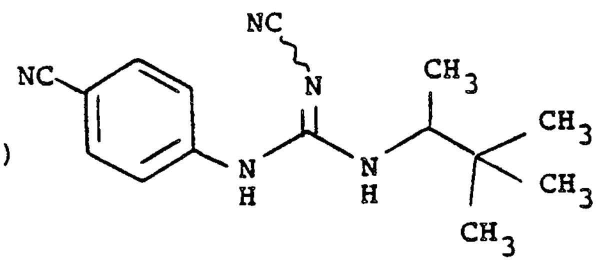 (E4)

Compound (E1) prepared as in Example 1, (±)-N-4- cyanophenyl-N′-1,2,2-trimethylprop-1-ylthiourea (2.61g), was mixed with N,N′-dicyclohexylcarbodiimide (4.12g), cyanamide (0.84g) and N,N-diisopropylethylamine (0.16g) in dry N,N-dimethylformamide (10 ml), and the mixture was stirred at room temperature for 48h. The solvent was evaporated in vacuo and the residue partitioned between ethyl acetate (150 ml) and water (200 ml). The aqueous phase was further extracted with ethyl acetate (100 ml), and the combined organic phase was washed with water (3x100 ml), then saturated sodium chloride solution (150 ml), and dried over anhydrous magnesium sulphate. Filtration and evaporation of the solvent in vacuo gave a yellow oil which partially solidified on standing. The mixture was triturated with ethyl acetate and the solid removed by filtration. Evaporation of the solvent gave an oil which was column chromatographed (Kieselgel 60: gradient elution: 0-1-% methanol-chloroform) to give a white foam (2.4g); recrystillisation from ethyl acetate-methanol gave the title compound as white needles (2g) having m.p. 194-5°C.
270 MHz $^1$H-nmr ($d_6$-DMSO) δ, 0.91 (s, 9H); 1.09 (d, J=7Hz, 3H); 3.78-3.95 (m, 1H); 7.31 (d, J=8Hz, 2H); 7.39 (d, J=9Hz, 1H); 7.75 (d, J=8Hz, 2H); 9.42 (s, 1H).

| | | | |
|---|---|---|---|
| Analysis. Found: | C, 66.89; | H, 6.96; | N, 25.95. |
| $C_{15}H_{19}N_5$ requires: | C, 66.89; | H, 7,11; | N, 26.00%. |

Example 5

(±)-N″-Cyano-N-3-cyanophenyl-N′-1,2,2-trimethylprop-1-ylguanidine (E5)

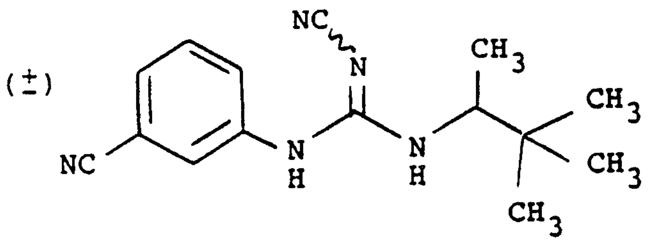

(E5)

The title compound was prepared analogously to the 4-cyanophenylguanidine of Example 4, starting from Compound (E2), prepared as in Example 2. Recrystallisation from ethyl acetate gave a white solid of m.p. 175-7°C.

270 MHz $^1$H-nmr ($d_6$-DMSO) δ, 0.89 (s, 9H); 1.07 (d, J=7Hz, 3H); 3.76-3.95 (m, 1H); 7.11 (d, J=10Hz, 1H); 7.40-7.66 (m, 4H); 9.22 (s, 1H).
Mass spectrum. Found: $M^+$ 269.1646; $C_{15}H_{19}N_5$ requires 269.1640.

| | | | |
|---|---|---|---|
| Analysis. Found: | C, 67.08; | H, 7.10; | N, 26.26. |
| $C_{15}H_{19}N_5$ requires | C, 66.89; | H, 7.11; | N, 26.00%. |

## Example 6

(±)-N″-Cyano-N-4-fluorophenyl-N′-1,2,2-trimethylprop-1-ylguanidine (E6)

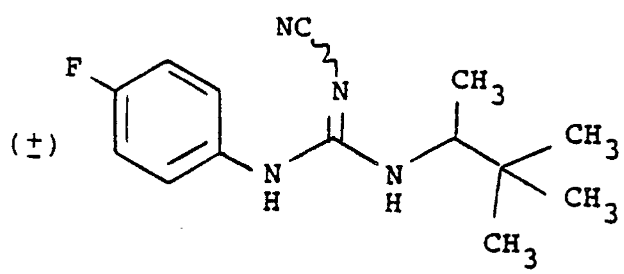

(E6)

Phosgene in toluene (4 ml, 1.93 M) was added to a stirred solution of Compound (E3), prepared as in Example 3, (±)-N-4-fluorophenyl-N′-1,2,2-trimethylprop-1-ylthiourea (1 g) in dry tetrahydrofuran (10 ml) at 0°C under an atmosphere of dry nitrogen. The solution was stirred for a further 5 h at 0°C, then the solvent was evaporated in vacuo. The residue was redissolved in dry tetrahydrofuran (10 ml) and cooled to 0°C under an atmosphere of dry nitrogen, and N,N-diisopropylethylamine (1.5g) was added to the cooled solution with stirring. The mixture was allowed to warm to room temperature and the solvent was then evaporated in vacuo. The residue was triturated under pentane (3x20 ml) and the combined pentane extractions evaporated in vacuo to give crude carbodiimide as an oil. The oil was dissolved in dry acetonitrile (5 ml) and cyanamide (0.33g) and N,N-diisopropylethylamine (50 mg) were added to this solution. The solution was stirred for 16 h at room temperature, then heated under reflux for 24 h. The solvent was evaporated in vacuo, and the residue partitioned between ethyl acetate (30 ml) and water (30 ml). The organic phase was then washed with 0.5 N hydrochloric acid (30 ml), water (2x30 ml), 5% (w/v) sodium hydrogencarbonate (30 ml) and saturated sodium chloride solution (30 ml). The organic phase was then dried over anhydrous sodium sulphate, filtered and the solvent evaporated in vacuo. The residual oil was column chromatographed (Kieselgel 60; eluting with chloroform) giving a colourless oil which solidified on standing. Recrystallisation from ethyl acetate gave the title compound as a white solid (0.62 g) having m.p. 161-3°C. 270 MHz $^1$H-nmr ($CDCl_3$) δ 0.83 (s, 9H); 1.03 (d, J=6Hz, 3H); 3.74-3.91 (m, 1H); 4.52 (d,

J=8Hz, 1H); 7.09-7.31 (m, 4H); 7.89 (s, 1H).

Example 7

N-4-Cyanophenyl-N′-t-butylthiourea (E7)

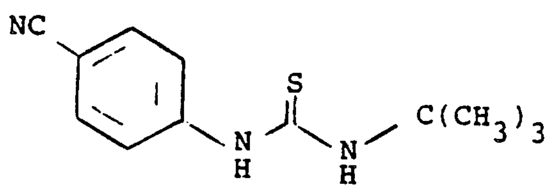

(E7)

The title compound was prepared in an analogous manner to that of Example 1, with the variation that t-butylamine was used instead of 2-amino-3,3-dimethylbutane. The title compound was obtained on recrystallisation from ethyl acetate as a white solid of m.p. 176°C.

270 MHz $^1$H-nmr (DMSO-$d_6$) δ 1.48 (s, 9H), 7.68 (d, J=8.5 Hz,2H), 7.75 (d, J=8.5Hz, 2H), 7.80 (s, 1H exchangeable $D_2O$), 9.66 (s, 1H, exchangeable $D_2O$).
Mass spectrum (EI) $M^+$ at M/Z 233.

Pharmacological Data

The activity of representative compounds of the invention was demonstrated as follows.

Bronchodilator Activity

Bronchodilation in vitro; guinea pig tracheal spiral preparations.

Male guinea pigs (300-600g) were stunned by a blow to the head and bled from the carotid artery. The trachea was exposed, dissected free of connective tissue, and transferred to oxygenated krebs solution at 37°C. Next, spirals (2 per trachea) were prepared by cutting the whole trachea spirally along its longitudinal axis and then dividing this spiral lengthwise. Each preparation was mounted, using silk thread, in a 10ml organ bath filled with krebs solution at 37°C and bubbled with 5% $CO_2$ with $O_2$. The resting tension of the preparations was set at 2g and changes in muscle tension were monitored isometrically by means of a UFI (2oz) force and displacement transducer (Ormed Ltd) connected to a Linseis pen recorder. All preparations were allowed to equilibrate for 60 minutes. During this equilibration period the preparations were washed by upward displacement at 15 minute intervals and, if necessary, the resting tension was readjusted to 2g using a mechanical micromanipulator system.

Once a steady resting tension had been obtained, the preparations were dosed cumulatively with the test compound ($10^{-8}$-$2x10^{-5}$M), and finally a maximum relaxation achieved by addition of $10^{-3}$M isoprenaline.

The fall in tension evoked by the test compound was expressed as a percentage of the total relaxation evoked in the presence of $10^{-3}$M isoprenaline. Appropriate concentration-relaxation curves were then constructed and values for potency ($IC_{50}$) were obtained.

(The composition of Krebs solution is: sodium chloride 118.07mM, sodium hydrogen carbonate 26.19mM, potassium chloride 4.68mM, potassium orthophosphate 1.18mM, magnesium sulphate septahydrate 1.8mM and calcium chloride 2.52mM;pH ca. 7.45.)

| Results | |
|---|---|
| Example No. | $IC_{50}$ (μM) |
| 3 | 5.9 |
| 4 | 3.0 |

## Claims

1. The use of a compound of formula (I), or a pharmaceutically acceptable salt thereof, or a pharmaceutically acceptable solvate thereof:

$$R_1-NH\overset{\overset{R_2}{\|}}{C}NHR_3$$

(I)

wherein:
$R_1$ is an aryl group substituted with up to three substituents selected from the group consisting of: CN, $NO_2$, $CF_3$, halogen, $C_{2-12}$ alkyl and $C_{2-12}$ alkyl carbonyl;
$R_2$ is S or NCN; and
$R_3$ is an alkyl group or an aryl group, for the manufacture of a medicament for the treatment of disorders of the respiratory system and/or for the treatment of cerebrovascular disorders and/or neuronal degenerative disorders.

2. A compound of formula (IA), or a salt thereof, or solvate thereof:

$$R_1-NH\overset{\overset{R_2}{\|}}{C}NHR_3$$

(IA)

characterised in that:
$R_1$ is an aryl group substituted with up to three substituents selected from the group consisting of: CN, $NO_2$, $CF_3$, halogen, $C_{2-12}$ alkyl and $C_{2-12}$ alkyl carbonyl;
$R_2$ is S or NCN; and
$R_3$ is a alkyl group or an aryl group,
provided that when $R_1$ is 4-tert. butyl-phenyl and $R_3$ is phenyl, then $R_2$ is NCN and provided that when $R_1$ represents 3- or 4-cyanophenyl and $R_3$ is 1,2,2-trimethylpropyl or when $R_1$ represents 2,6-dichlorophenyl and $R_3$ is tert. butyl, then $R_2$ is S.

3. A compound according to claim 2, wherein $R^2$ represents S.

4. A compound, according to claim 2 or claim 3, wherein $R_1$ is monosubstituted.

5. A compound, according to any one of claims 2 to 4, wherein $R_1$ is 3-cyanophenyl, 4-cyanophenyl, 3-fluorophenyl or 4-fluorophenyl.

6. A compound according to any one of claims 2 to 5, wherein $R_3$ is a branched $C_{4-6}$ or a branched $C_{5-9}$ alkyl group:

7. A compound according to any one of claims 2 to 6, wherein $R_3$ is a tert. butyl or a 1,2,2-trimethylpropyl group.

8. A compound according to claim 2 of formula (IB):

$$R_1-NH\overset{R_2}{\overset{\|}{C}}NHR_3$$

(IB)

wherein:
$R_1$ is phenyl monosubstituted with CN, $NO_2$, $CF_3$, halogen, $C_{2-6}$ alkyl or a $C_{2-6}$ alkyl carbonyl group;
$R_2$ is S or NCN; and
$R_3$ is a $C_{1-9}$ alkyl group or phenyl, provided that when $R_1$ is 4-tert. butyl-phenyl and $R_3$ is phenyl, then $R_2$ is NCN and provided that when $R_1$ represents 3- or 4-cyanophenyl and $R_3$ is 1,2,2-trimethylpropyl or when $R_1$ represents 2,6- dichlorophenyl and $R_3$ is tert.butyl, then $R_2$ is S.

9. A compound according to claim 2, selected from the group consisting of:
(±)-N-4-cyanophenyl-N′-1,2,2-trimethylprop-1-ylthiourea;
(±)-N-3-cyanophenyl-N′-1,2,2-trimethylprop-1-ylthiourea;
(±)-N-4-fluorophenyl-N′-1,2,2-trimethylprop-1-ylthiourea;
(±)-N″-cyano-N-4-fluorophenyl-N′-1,2,2-trimethylprop-1-ylguanidine; and
N-4-cyanophenyl-N′-t-butylthiourea; or a salt thereof, or a solvate thereof.

10. A compound according to claim 2, being (±)-N-4-cyanophenyl-N′-1,2,2-trimethylprop-1-ylthiourea; or a salt thereof, or a solvate thereof.

11. A process for the preparation of a compound of formula (IA), characterised in that the process comprises:

a) for compounds of formula (IA) wherein $R_2$ is S, reacting a compound of formula (II)

$R_1' - N = C = S$ (II)

with a compound of formula (III)

$R_3'NH_2$ (III)

where $R_1'$ and $R_3'$ are respectively $R_1$ and $R_3$ or groups convertible thereto, to form a compound of formula (IC)

$$R_1' - \underset{H}{N} - \overset{S}{\overset{\|}{C}} - \underset{H}{N} - R_3'$$

(IC)

or

b) for compounds of formula (IA) wherein $R_2$ is SCN, reacting a compound of formula (IV)

$R_1' - N = C = N - R_3'$ (IV)

with cyanamide, to form a compound of formula (ID)

$$R_1' - \underset{H}{N} - \overset{NCN}{\overset{\|}{C}} - \underset{H}{N} - R_3'$$

(ID)

and thereafter if required carrying out one or more of the following optional steps:

(i) converting $R_1'$ or $R_3'$ to $R_1$ and $R_3'$ respectively;
(ii) preparing a salt of the compound of formula (I);
(iii) preparing a solvate of a compound of formula (I) or a salt thereof.

12. A compound of formula (IA), or a pharmaceutically acceptable salt, or a pharmaceutically acceptable solvate thereof, for use as an active therapeutic substance.

13. A pharmaceutical composition comprising a therapeutically effective amount of a compound of formula (IA), or a pharmaceutically acceptable salt, or a pharmaceutically acceptable solvate thereof, and a pharmaceutically acceptable carrier.

Claims for the following Contracting State: ES

1. A process, for preparing a compound of formula (IA), or a salt thereof, or solvate thereof:

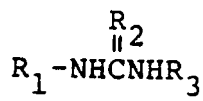

(IA)

wherein:
$R_1$ is an aryl group substituted with up to three substituents selected from the group consisting of: CN, $NO_2$, $CF_3$, halogen, $C_{2-12}$ alkyl and $C_{2-12}$ alkyl carbonyl;
$R_2$ is S or NCN; and
$R_3$ is a alkyl group or an aryl group,
provided that when $R_1$ is 4-tert. butyl-phenyl and $R_3$ is phenyl, then $R_2$ is NCN and provided that when $R_1$ represents 3- or 4-cyanophenyl and $R_3$ is 1,2,2-trimethylpropyl or when $R_1$ represents 2,6-dichlorophenyl and $R_3$ is tert. butyl, then $R_2$ is S:
characterised in that the process comprises:

a) for compounds of formula (IA) wherein $R_2$ is S, reacting a compound of formula (II)

$R_1' - N = C = S$ (II)

with a compound of formula (III)

$R_3'NH_2$ (III)

where $R_1'$ and $R_3'$ are respectively $R_1$ and $R_3$ or groups convertible thereto, to form a compound of formula (IC)

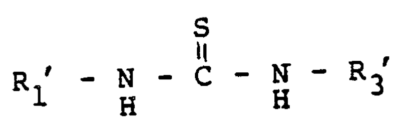

(IC)

or

b) for compounds of formula (IA) wherein $R_2$ is SCN, reacting a compound of formula (IV)

$R_1' - N = C = N - R_3'$ (IV)

with cyanamide, to form a compound of formula (ID)

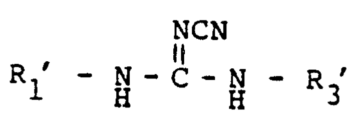

(ID)

and thereafter if required carrying out one or more of the following optional steps:

(i) converting $R_1'$ or $R_3'$ to $R_1$ and $R_3'$ respectively;

(ii) preparing a salt of the compound of formula (I);

(iii) preparing a solvate of a compound of formula (I) or a salt thereof.

2. A process according to claim 1, characterised in that $R^2$ represents S.

3. A process, according to claim 1 or claim 3, characterised in that $R_1$ is monosubstituted.

4. A process, according to any one of claims 1 to 3, characterised in that $R_1$ is 3-cyanophenyl, 4-cyanophenyl, 3-fluorophenyl or 4-fluorophenyl.

5. A process according to any one of claims 1 to 4, characterised in that $R_3$ is a branched $C_{4-6}$ or a branched $C_{5-9}$ alkyl group.

6. A process according to any one of claims 1 to 5, characterised in that $R_3$ is a <u>tert.</u> butyl or a 1,2,2-trimethylpropyl group.

7. A process according to claim 1 characterised in that
$R_1$ is phenyl monosubstituted with CN, $NO_2$, $CF_3$, halogen, $C_{2-6}$ alkyl or a $C_{2-6}$ alkyl carbonyl group;
$R_2$ is S or NCN; and
$R_3$ is a $C_{1-9}$ alkyl group or phenyl, provided that when $R_1$ is 4-<u>tert.</u> butyl-phenyl and $R_3$ is phenyl, then $R_2$ is NCN and provided that when $R_1$ represents 3- or 4-cyanophenyl and $R_3$ is 1,2,2-trimethylpropyl or when $R_1$ represents 2,6- dichlorophenyl and $R_3$ is <u>tert.</u> butyl, then $R_2$ is S.

8. A process according to claim 1, characterised in that the compound of formula (IA) is selected from the group consisting of:
(±)-N-4-cyanophenyl-N′-1,2,2-trimethylprop-1-ylthiourea;
(±) -N-3-cyanophenyl-N′-1,2,2-trimethylprop-1-ylthiourea;
(±)-N-4-fluorophenyl-N′-1,2,2-trimethylprop-1-ylthiourea;
(±)-N″-cyano-N-4-fluorophenyl-N′-1,2,2-trimethylprop-1-ylguanidine; and
N-4-cyanophenyl-N′-t-butylthiourea; or a salt thereof, or a solvate thereof.

9. A process according to claim 1, characterised in that the compound of formula (IA) is (±)-N-4-cyanophenyl-N′-1,2,2-trimethylprop-1-ylthiourea; or a salt thereof, or a solvate thereof.

10. The use of a compound of formula (I), or a pharmaceutically acceptable salt thereof, or a pharmaceutically acceptable solvate thereof:

$$R_1\text{-NH}\overset{\overset{\displaystyle R_2}{\|}}{\text{C}}\text{NHR}_3 \qquad \text{(I)}$$

wherein:
$R_1$ is an aryl group substituted with up to three substituents selected from the group consisting of: CN, $NO_2$, $CF_3$, halogen, $C_{2-12}$ alkyl and $C_{2-12}$ alkyl carbonyl;
$R_2$ is S or NCN; and
$R_3$ is an alkyl group or an aryl group, for the manufacture of a medicament for the treatment of disorders of the respiratory system and/or for the treatment of cerebrovascular disorders and/or neuronal degenerative disorders.